# FASCICULE DE BREVET EUROPEEN

(11) **EP 0 894 768 B1**
(45) Date de publication et mention de la délivrance du brevet: **04.10.2001**
(21) Numéro de dépôt: 98401902.6
(22) Date de dépôt: 27.07.1998
(51) Int. Cl.: C01B 39/44, B01J 29/65, C07C 5/27, C07C 4/06

(54) **Procédé de préparation d'une zéolithe de type ferrierite utilisable comme catalyseur d'isomérisation d'une oléfine linéaire en isooléfine ou comme catalyseur d'hydrocraquage et d'hydroisomérisation des paraffines**
Verfahren zur Herstellung eines Zeoliths des Ferrierit-Typs, Verwendbar als Isomerisierungskatalysator eines linearen Olefins zu Isoolefin oder als Hydrocrackungs- und Hydroisomerisierungskatalysator von Paraffinen
Process for producing a ferrierite-type zeolite usable as catalyst for isomerizing a linear olefin to an isoolefin or as catalyst for hydrocracking and hydroisomerizing paraffins

(30) Priorité: 31.07.1997 FR 9709768
(43) Date de publication de la demande: 03.02.1999
(73) Titulaire: TOTAL RAFFINAGE DISTRIBUTION S.A., 92800 Puteaux (FR)
(72) Inventeur: Szabo, Georges, 76290 Montivilliers (FR); Decker, Sébastien, 76600 Le Havre (FR); Meriaudeau, Paul, 01160 Pont d'Ain (FR); Vu, Anh Tuan, 69100 Villeurbanne (FR)
(74) Mandataire: Jolly, Jean-Pierre

(56) Documents cités:
- EP-A- 0 523 838
- WO-A-96/40587
- US-A- 3 933 974
- US-A- 4 650 654

## Description

La présente invention concerne un procédé de préparation d'une zéolithe synthétique de structure ferrierite (FER), cristallographiquement pure, à taille de cristaux homodispersée, sans l'emploi d'agent structurant, et son utilisation comme catalyseur acide, en particulier pour l'isomérisation squelettale d'oléfines linéaires telles que les butènes ou les pentènes, et comme catalyseur d'hydrocraquage et d'hydroisomérisation des paraffines à longue chaîne, linéaires ou peu ramifiées, issues notamment de la réaction de Fischer-Tropsch à partir du gaz de synthèse (CO+H₂).

Il est connu, dans la technique, que des matériaux dénommés zéolithes, qu'ils soient naturels ou synthétiques, possédent des propriétés catalytiques pour divers types de conversion des hydrocarbures. Typiquement, ces matériaux sont des alumino-silicates cristallins poreux possédant une structure cristalline définie, pourvue d'un nombre important de très petits canaux ou pores de taille uniforme, adaptés pour l'absorption de molécules de dimensions spécifiques. Parmi ces zéolithes du type alumino-silicates naturels, figure la ferrierite.

Une grande variété de procédés d'obtention de zéolithes synthétiques a été décrite dans l'art antérieur, en particulier la zéolithe A (brevet US N° 2 882 243), la zéolithe Y (brevet US N° 3 130 007) et la zéolithe dite ZSM-5 (brevet US N° 3 702 886).

De même, le brevet US N° 4 016 245 décrit la synthèse d'une zéolithe cristalline à base d'alumino-silicate, dénommée ZSM-35, possédant une structure cristallographique très proche de celle de la ferrierite et pouvant servir de catalyseur pour la conversion des hydrocarbures. Toutefois, cette synthèse met toujours en jeu un composé organo-azoté tel que de l'éthylènediamine ou de la pyrrolidine, qui agit comme agent structurant orientant la synthèse vers une structure déterminée. Cette zéolithe possède un réseau orthorhombique comprenant deux séries de canaux unidimensionnels se coupant perpendiculairement, une série étant composée de canaux à 10 "atomes T " (atomes tétraédriques de Si, Al) de section sensiblement elliptique (4,2 x 5,4 Å), et l'autre de canaux à 8 "atomes T " (3,5 x 4,8 Å).

D'autres brevets revendiquent un procédé de fabrication de ce matériau utilisant divers agents structurants tels que des alkylpyridines (brevet US N° 4 251 499) ou des alkylpipéridines (brevet US N° 4 795 623) ou d'autres agents.

Il est connu, par la demande de brevet EP 0523 838 (Lyondell), d'utiliser dans un procédé d'isomérisation squelettale d'oléfines linéaires en isooléfines, telles que le butène-1 ou le pentène-1, un catalyseur du type zéolithe ayant une structure et une dimension de pores adaptées pour réduire les réactions parasites et le cokage. Ce document décrit de nombreux exemples de zéolithes, dont les ferrierites, mais ne mentionne pas leur procédé de fabrication.

Une étude récente de WEN QING XU et al., publiée dans J. PHYS.CHEM. 1995, Vol.99, 9443-9451, qui a comparé les structures de différentes zéolithes ZSM-35 du type ferrierite, synthétisées respectivement avec un agent structurant (pyrrolidine) et sans un tel agent, ayant un rapport Si/Al compris entre 5,7 et 9,2, montre que la première catégorie présente des particules dont la taille a une répartition uniforme, alors que la taille des cristallites de la seconde catégorie est très hétérogène, variant de quelques µm à 100 µm. En outre, les zéolithes de la première catégorie présentent une cristallinité plus élevée et comportent moins de mésoporosité que les zéolites obtenues sans agent structurant. Il en résulte une augmentation de la sélectivité dans l'isomérisation squelettale du butène linéaire en isobutène pour les zéolithes synthétisées avec agent structurant.

Cette étude précise que la synthèse réussie de zéolithes ZSM-35 du type ferrierite, sans utilisation d'agent structurant organique,nécessite d'avoir des gels précurseurs ayant une plage étroite du rapport Si/Al (égal environ à 9) et des conditions d'agitation pendant le traitement en autoclave.

Le but de la présente invention est donc de proposer un procédé permettant d'obtenir, sans utilisation d'agent structurant, une zéolithe du type ferrierite, qui est très bien cristallisée, avec une taille de cristaux faible et monodispersée, et qui possède de bonnes propriétés comme catalyseur dans les réactions acides telles que l'isomérisation des butènes en isobutènes ou des pentènes en isopentènes, et comme catalyseur d'hydrocraquage et d'hydroisomérisation des paraffines à longue chaîne, linéaires ou peu ramifiées, issues notamment de la réaction de Fischer-Tropsch à partir du gaz de synthèse (CO+H₂).

Plus particulièrement, l'invention concerne un procédé de préparation d'une zéolithe du type ferrierite, à taux de cristallinité élevé et ayant une composition au stade anhydre exprimée par la formule Mₓ (AlO₂)ₓ (SiO₂)_{y}, M étant un cation d'un métal alcalin tel que Na ou K, ou un mélange des deux, caractérisé en ce qu'il comprend une étape d'obtention d'un gel, par addition d'acide borique à une solution aqueuse de sulfate d'aluminium et mélange avec une solution aqueuse contenant des oxydes de Na et/ou K et de Si, sans emploi d'agent structurant.

De préférence, le mélange des solutions se fait sous agitation, avec acidification jusqu'à pH 8.

Selon un mode de mise en oeuvre préféré de procédé, le gel obtenu est lavé à l'eau distillée .

Avantageusement, une solution contenant des hydroxydes de métaux alcalins est ajoutée sous agitation au mélange des solutions.

Le gel obtenu peut être soumis à une étape de vieillissement, suivie d'une étape de cristallisation en autoclave, sous agitation et à chaud, pendant plusieurs heures, et le produit cristallisé obtenu peut être lavé à l'eau et séché à chaud.

Selon un mode de mise en oeuvre particulier du procédé conforme à l'invention, les étapes d'obtention du gel et d'adjonction des hydroxydes de métaux alcalins sont conduites à une température comprise entre 40 et 90°C, tandis que l'étape de vieillissement du gel est conduite à une température comprise entre 25 et 80°C, pendant une durée de 6 à 72 heures.

Selon une variante de mise en oeuvre, l'étape de cristallisation s'effectue à pression autogène, à une température comprise entre 150 et 200°C et pendant 2 à 72 heures.

Selon une autre variante de mise en oeuvre, l'étape de cristallisation est suivie d'une calcination, d'une imprégnation avec une solution d'un sel de platine, puis d'une évaporation, d'un séchage et d'une autre calcination.

Une caractéristique importante du procédé conforme à l'invention réside dans le fait que les atomes de bore ne restent pas dans la structure cristalline, mais se retrouvent à la sortie de l'autoclave dans la phase liquide.

L'étape de vieillissement a également une influence importante sur la cristallinité de la zéolithe obtenue, de même que les conditions du traitement en autoclave, notamment sa durée (synthèse hydrothermale).

Un avantage important du procédé de préparation de la zéolithe selon l'invention, réside dans la suppression de l'étape usuelle de calcination à 500°C, qui est nécessaire lorsqu'on utilise un agent structurant organique.

La taille moyenne des cristaux de la zéolithe obtenue par le procédé conforme à l'invention est comprise entre 0,1 et 1µm.

D'autres caractéristiques et avantages de l'invention apparaîtront à la lecture de la description des exemples suivants, qui illustrent différents procédés de préparation, et notamment des exemples 3 à 6 et 9, qui se rapportent à des modes de mise en oeuvre de l'invention, utilisant de l'acide borique.

### EXEMPLE 1

Cet exemple concerne un procédé de préparation connu d'une zéolithe du type ferrierite.

On prépare une solution A de silicate de sodium (23 g de silicate de sodium à 26% de SiO₂, 8% de Na₂O) avec 70 g d'eau. La solution est chauffée à 60°C, pendant 20 minutes. Une solution B contenant 3,15 g de sulfate d'aluminium [Al₂ (SO₄)₃, 14H₂O] avec 50 g d'eau est chauffée pendant 20 minutes à 70°C sous agitation, jusqu'à l'obtention d'une solution claire.

La solution B est ajoutée à la solution A sous agitation et un précipité se forme.

On ajoute 20 g d'eau à ce mélange, sous agitation, puis, goutte à goutte, de l'acide sulfurique pur, jusqu'à ce que le pH de 8 soit atteint.

Au mélange visqueux résultant, on ajoute 20 g d'eau et l'on chauffe sous agitation pendant 40 minutes. Le gel obtenu est ensuite lavé à l'eau distillée et centrifugé, l'opération étant répétée 8 fois.

Au gel ainsi préparé, on ajoute, sous agitation, un mélange de 0,4 g de NaOH et 0,4 g de KOH dans 10 g d'eau, le tout étant chauffé à 80°C sous agitation pendant 1 heure. Le poids final du gel obtenu est de 54 à 55 g.

La cristallisation s'opère en transférant le gel dans un autoclave, qui est scellé, agité et chauffé à 180°C, pendant 3 jours, (vitesse de rotation 300 tr/mn).

La mise en forme de 22 g du produit obtenu est faite en mélangeant ce dernier avec 5,45 g d'Alumine Pural SB et 31 ml d'eau. Après formation de la pâte, on extrude à travers une filière de 1,8 mm de diamètre. Les extrudés obtenus sont séchés à l'air à 120°C pendant une nuit, puis calcinés sous air en augmentant la température régulièrement depuis la température ambiante jusqu'à 500°C à raison de 1,5°C /min.

### EXEMPLE 2

Cet exemple est dérivé de l'exemple 1.

On utilise une solution A, contenant 23 g de silicate de sodium (26% de SiO₂) et 150 g d'eau, qui est chauffée à 60°C, pendant 20 minutes.

La solution B contient 3,15 g d'Al₂ (SO₄)₃, 14 H₂ O avec 150 g d'eau. La solution B est chauffée à 70°C, pendant 20 minutes. Les étapes suivantes sont identiques à celles de l'exemple 1.

### EXEMPLE 3

Cet exemple illustre une mise en oeuvre du procédé conforme à l'invention.

On prépare une solution A identique à celle de l'exemple 1.

Une solution B contenant 1,24 g d'acide borique B(OH)₃, 2,96 g d'Al₂(SO₄)₃,14 H₂O et 50 g d'eau est préparée sous agitation à 70°C, pendant 20 minutes. La solution B est ajoutée à la solution A sous agitation. Un précipité se forme, le pH étant voisin de 10.

20 g d'eau sont ajoutés sous agitation. Après 20 minutes, on ajoute au goutte à goutte de l'acide sulfurique, jusqu'à ce que le pH redescende à 8. A ce stade, le mélange est très visqueux et l'on ajoute 20 g d'eau, en maintenant la température à 70°C, sous agitation pendant 40 minutes.

Le gel est ensuite lavé 8 fois à l'eau distillée (200 g à chaque fois).

A ce gel on ajoute une solution C (0,4 g de NaOH, 0,4 g de KOH et 10 g d'eau) sous agitation à 80°C pendant 1 heure. Le poids final du gel obtenu est de 54 à 55 g.

La cristallisation est assurée en autoclave, à pression autogène pendant 3 jours à 180°C, sous agitation (300 tr/mn). Ensuite, on procède à une calcination qui est faite sous flux d'air, en augmentant la température depuis la température ambiante jusqu'à 500 °C à raison de 1,5 °C/min. Cette dernière température est maintenue pendant 12 heures.

Puis, on procède à l'activation de la ferrierite la substitution du Na⁺ et du K' se fait par 3 échanges à reflux consécutifs en présence de NH₄NO₃ 1M (250 ml). Ces échanges sont suivis d'un rinçage à l'eau distillée, d'un séchage pendant une nuit à 120°C puis d'une autre calcination telle que celle présentée ci-dessus mais en maintenant la température finale (500°C) pendant seulement 4h.

La mise en forme est effectuée comme dans l'exemple 1.

### EXEMPLE 4

On opère comme dans l'exemple 3, mais, avant la cristallisation en autoclave, le gel est vieilli pendant 2 jours à 25°C.

Les étapes de cristallisation, de calcination et d'activation sont identiques à celles de l'exemple 3.

La mise en forme est effectuée comme dans l'exemple 1.

### EXEMPLE 5

Cet exemple concerne également une modification de l'exemple 3.

On opère comme dans cet exemple 3, mais, avant la cristallisation, on ajoute 1% en poids de germes de la zéolithe synthétisée dans l'exemple 3. La cristallisation est effectuée pendant 2 jours à 180°C ou pendant 1 jour à 195°C.

La mise en forme est effectuée comme dans l'exemple 1.

### EXEMPLE 6

On opère comme dans l'exemple 3, mais en diminuant la teneur en aluminium.

On prépare une solution A identique à celle de l'exemple 3.

On prépare une solution B contenant 2,46g de Al₂ (SO₄)₃, 14H₂O et 1,24 g de B(OH)₃ dans 50 g d'eau. La solution B est chauffée sous agitation à 70°C pendant 20 minutes.

Les étapes suivantes sont identiques à celles décrites dans l'exemple 3.

### EXEMPLE 7

La ferrierite de cet exemple est préparée en utilisant, de façon usuelle, un agent structurant : 10,87 g de pyrrolidine sont ajoutés à 48,4 g de silica-sol (type AS40, 40% de SiO₂) sous agitation à température ambiante. Après 15 minutes d'agitation, une solution homogène A est obtenue. Une solution B, constituée de 0,5 g de NaOH, 3g de NaAlO₂ et 136 g d'eau, est préparée à 70°C, sous agitation pendant 40 minutes, afin d'obtenir une solution claire.

La solution B est alors ajoutée à la solution A sous agitation, pour obtenir un gel homogène qui est agité pendant 1 heure.

Ce gel est transféré dans un autoclave que l'on ferme et que l'on chauffe à 180°C pendant 22 jours.

La mise en forme est effectuée comme dans l'exemple 1.

### EXEMPLE 8

Cet exemple concerne également une préparation effectuée en utilisant un agent structurant.

29 g de silicate de Na (26% de SiO₂) sont dissous dans 70 g d'eau, la solution A étant chauffée sous agitation à 60°C pendant 20 minutes.

Une solution B (2,97g de Al₂ (SO₄)₃, 14H₂O + 50 g d'eau) est chauffée à 70 °C pendant 20 minutes.

La solution B est ajoutée à la solution A sous agitation. Un précipité se forme. On ajoute 20 g d'eau au gel résultant, sous agitation, puis l'on ajoute goutte à goutte de l'acide sulfurique pour que le pH redescende à 8.

20 g d'eau sont ajoutés sous agitation pendant 40 minutes à 70°C.

Le gel est récupéré et lavé 8 fois à l'eau.

On ajoute au gel lavé 3 g de pyrrolidine, sous agitation, puis 0,47 g de NaOH (sous forme de poudre) sous agitation, pendant 1 heure, à température ambiante.

Le gel est ensuite transféré dans un autoclave scellé, chauffé à 180°C sous agitation pendant 3 jours (vitesse de rotation 300 tr/mn).

La mise en forme est effectuée comme dans l'exemple 1.

### EXEMPLE 9

Cet exemple illustre une mise en oeuvre du procédé conforme à l'invention.

On prépare une solution A identique à celle de l'exemple 1.

Une solution B contenant 1,24 g d'acide borique B(OH)₃, 2,96 g d'Al₂(SO₄)₃, 14 H₂O et 50 g d'eau est préparée sous agitation à 70°C, pendant 20 minutes. La solution B est ajoutée à la solution A sous agitation. Un précipité se forme, le pH étant voisin de 10.

20 g d'eau sont ajoutés sous agitation. Après 20 minutes, on ajoute au goutte à goutte de l'acide sulfurique, jusqu'à ce que le pH redescende à 8. A ce stade, le mélange est très visqueux et l'on ajoute 20 g d'eau, en maintenant la température à 70°C, sous agitation pendant 40 minutes.

Le gel est ensuite lavé 8 fois à l'eau distillée (200 g à chaque fois).

A ce gel on ajoute une solution C (0,4 g de NaOH, 0,4 g de KOH et 10 g d'eau) sous agitation à 80°C pendant 1 heure. Le poids final du gel obtenu est de 54 à 55 g.

La cristallisation est assurée en autoclave, à pression autogène pendant 3 jours à 180°C, sous agitation (300 tr/mn).

Ensuite, on procède à une calcination qui est faite sous flux d'air, en augmentant la température depuis la température ambiante jusqu'à 500 °C à raison de 1,5 °C/min. Cette dernière température est maintenue pendant 12 heures.

Puis, la ferrierite est activée de la manière suivante : on procède à la substitution du Na⁻ et du K⁻ en effectuant 3 échanges à reflux consécutifs en présence de NH₄NO₃ 1M (250 ml). Ces échanges sont suivis d'un rinçage à l'eau distillée, d'un séchage pendant une nuit à 120°C puis d'une calcination telle que présentée ci-dessus mais en maintenant la température finale (500°C) pendant 4h.

On dépose ensuite du platine à l'aide d'une solution composée de 110 ml d'eau permutée et 0,324 g de Pt(NH₃)₄Cl₂H₂O mise en contact avec 22,4 g de ferrierite. Un contact statique de 16 h est imposé avant d'évacuer la phase aqueuse à l'aide d'un évaporateur rotatif suivi d'un séchage pendant un nuit à 120°C. Enfin, une calcination du précurseur formé est effectuée sous air, en augmentant la température de manière linéaire depuis la température ambiante jusque 300°C. Cette température est maintenue pendant 1 h avant de poursuivre la chauffe jusqu'à 500°C.

Le catalyseur est ensuite mis en forme comme indiqué dans l'exemple I.

Il est à noter que l'étape de cristallisation du procédé conforme à l'invention, ou synthèse hydrothermale, peut se faire en utilisant d'autres sources d'énergie et en particulier les microondes, qui permettent de réduire la durée de cette étape, tout en restant à la même température.

Tous les matériaux synthétisés obtenus dans ces exemples présentent la structure cristalline de la ferrierite et leurs caractéristiques cristallographiques sont rassemblées dans le Tableau I ci-après.

**TABLEAU 1**

| Exemple | 1 | 2 | 3 | 4 | 5 | 6 | 7 | 8 |
|---|---|---|---|---|---|---|---|---|
| Si/Al (analyse chimique) | 9,2 | 9,1 | 10,8 | 11 | 10,7 | 9,3 | 8,8 | 8,9 |
| Si/Al (RMN) a) | 9,8 | 9,9 | 11,8 | 11,5 | 10 | 10,5 | / | 9 |
| Taille des cristaux (MEB) en *µ*m b) | 0,5 à 1 | 0,5 à 1 | 0,5 à 1 | 0,5 à 1 | 0,5 à 1 | 0,5 à 1 | 20 à 30 | 1 à 2 |
| Hauteur de la raie RX pour 2θ = 9,4 degrés c) | 53 | 52 | 60 | 100 | 40 | 55 | 20 à 30 | 80 |

a) valeur mesurée à partir du spectre de ²⁹Si.
b) estimée par microscopie électronique à balayage MEB(moyenne).
c) la hauteur est fixée à 100 pour l'échantillon le mieux cristallisé.

Si l'on admet, comme l'expose SZOSTAK R., dans MOLECULAR SIEVES, Editeur VAIN NOSTRAND REINHOLD, NEW YORK, 1989 p 290, que la hauteur des raies de diffraction (pics) de RX est en relation directe avec le degré de cristallisation, pour des cristaux dont la taille est supérieure à 0,3 µm, il apparaît, en comparant les hauteurs des pics à 2ϑ = 9,45°, dans le Tableau 1, que les matériaux des Exemples 1 à 8 sont bien cristallisés (se référer à l'ouvrage " Collection of simulated XRD powder patterns for zeolites" " de TRACYHIGGINS and VON BALLMOOS, Editeur ELSEVIER 1996). Cependant, le degré de cristallinité le plus élevé est obtenu pour l'Exemple 4.
En outre, l'examen des clichés des échantillons de zéolithe correspondant aux divers exemples de préparation, obtenus par microscopie électronique à balayage, a permis d'évaluer la taille des cristaux de ces échantillons, qui est indiquée dans ce Tableau I.

### TESTS CATALYTIQUES DES FERRIERITES OBTENUES DANS L'ISOMÉRISATION DU BUTÈNE-1 EN ISOBUTÈNE

Tous les échantillons de zéolithe des Exemples 1, 3 à 5 et 8, sont testés dans les mêmes conditions expérimentales :
- Poids de catalyseur: 0,1-0,2 g,
- T : 400°C,
- pph (poids de butène-1/poids de catalyseur/heure) : 5 h⁻¹,
- pression partielle du butène-1:26 kPa (complément à la pression atmosphérique,soit 101 kPa, par N₂).

Au cours de la réaction d'isomérisation, un équilibre est atteint entre les butènes linéaires, butène-1 et butène-2 cis et trans.

Les progrès de l'isomérisation, en fonction du temps de réaction sont rapportés dans le Tableau II ci-après, dans lequel sont spécifiés :
- le rendement en isobutène, c'est-à-dire le rapport du poids d'isobutène obtenu à celui du butène-1 initial, exprimé en %,
- la conversion des butènes linéaires, c'est-à-dire le rapport de la différence entre le poids initial en butène-1 et le poids final en butène-1 et butène-2, au poids initial de butène-1, exprimé en %,
- la sélectivité en isobutène, c'est-à-dire le rapport du poids d'isobutène obtenu au poids de butène-1 converti, exprimé en % .

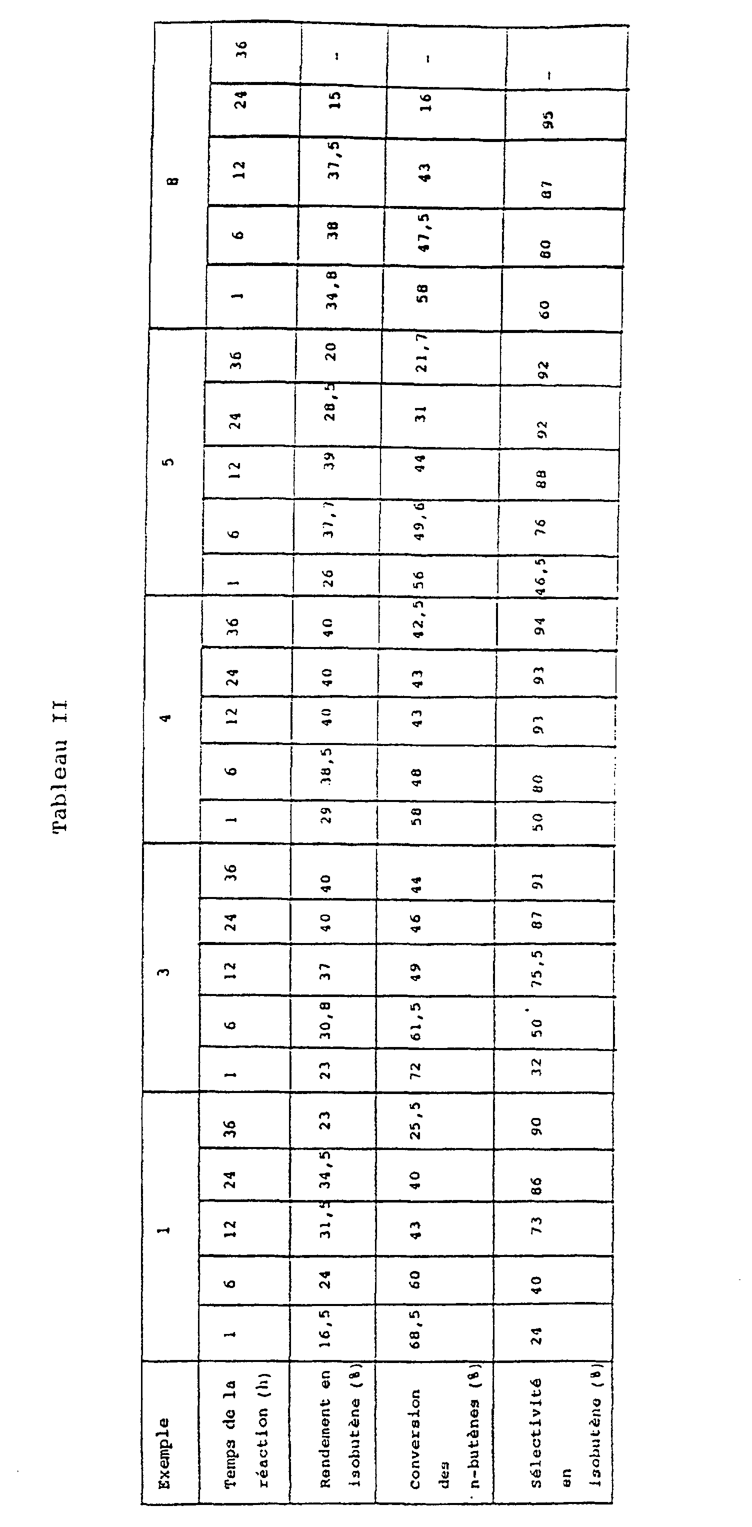

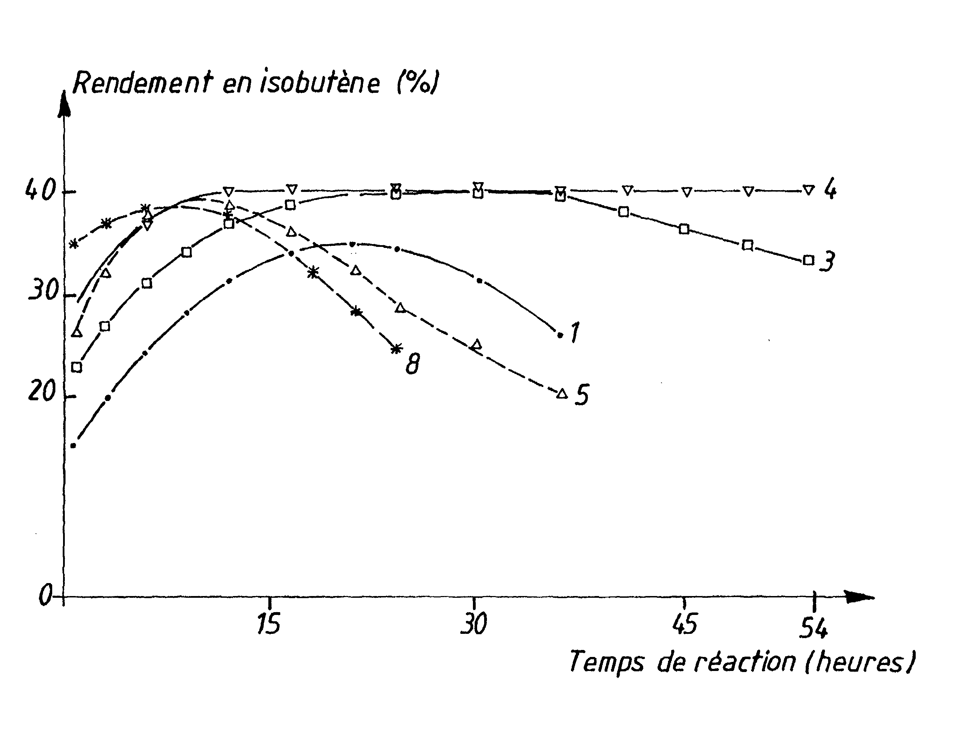

Le graphique de la figure 1 annexée illustre le rendement en isobutène (en %) en fonction du temps de réaction (en heures), pour les exemples du Tableau II. On constate que ce sont les catalyseurs des exemples 3 et 4, selon l'invention, qui sont les plus performants, notamment en rendement en isobutène au-delà de 12 heures de réaction, et qu'ils conservent cette efficacité bien après 36 heures . Les essais effectués ont également montré que les zéolithes obtenues sans agent structurant possèdent une meilleure activité d'isomérisation que les catalyseurs classiques utilisés dans ce domaine.

### TESTS EFFECTUES AVEC LE CATALYSEUR DE L'EXEMPLE 9 (Pt/Ferrierite) DANS DES REACTIONS D'HYDROCRAQUAGE ET D'HYDROISOMERISATION

L'activité de ce catalyseur dans les réactions d'hydrocraquage/hydroisomérisation a été testée sur deux charges : le n-dodécane et le n-hexadécane. Les conditions de réaction sont :

| | |
|---|---|
| Température | 250 à 300°C |
| Pression | 20 à 50 bars = 2 à 5 MPa |
| H₂/HC (charge) | 3 à 6 |
| PPH | de 0,75 à 2h⁻¹ |

On place 7 g de catalyseur dans le réacteur pour chaque test.

Avant toute réaction, le catalyseur est réduit in-situ dans le réacteur sous un débit de 6 1h⁻¹ d'hydrogène à 30 bars (3 MPa) et à une température de 500°C pendant 2 heures. La charge est séchée au maximum sur zéolithe 4A avant d'être mélangée à l'hydrogène et mise en contact avec le catalyseur.

### 1- Transformation du n-hexadécane

Comme l'illustrent les résultats présentés dans le tableau III ci-dessous, la transformation de l'hexadécane sur le catalyseur de l'exemple 9 contenant 0,8% en poids de Pt par rapport au substrat ferrierite aboutit à la formation de produits dont la nature dépend étroitement des conditions opératoires utilisées. En effet, ce catalyseur peut, soit favoriser l'isomérisation de la charge, soit favoriser son hydrocraquage.

La température est bien entendu un paramètre important qui intervient à la fois sur l'activité et la sélectivité des catalyseurs. On constate l'augmentation de la conversion du n-C₁₆ avec l'augmentation de la température de réaction, pour atteindre 100% à 300°C (30 bars, H₂/HC=3, pph=1). Cette augmentation de la conversion par effet de la température entraîne une augmentation du pouvoir hydrogénolysant du catalyseur au détriment de l'isomérisation du réactif. De plus; la formation de composés légers (C₄) est favorisée par une augmentation de la température de réaction. Compte tenu des résultats présentés dans le tableau III, une température proche de 280°C semble être la plus intéressante pour obtenir une conversion élevée sans trop favoriser la formation de composés légers (C₄).

**Tableau III**

| **Hydrocraquage du n-C**_{**16**} **sur Pt/Ferrierite** | | | | | | | |
|---|---|---|---|---|---|---|---|
| **T (°C)** | **P (bar)** | **H**_{**2**}**/HC** | **pph h**^{**-1**} | **Conversion (%)** ^{**(1)**} | **Rendement iC16 (%)**^{**(2)**} | **Rendement C5-C11 (%)**^{**(2)**} | **Rendement C12-C15 (%)** |
| ***275*** | ***20*** | ***3*** | ***1*** | ***90,2*** | ***45,46*** | ***10,01*** | ***9, 73*** |
| 275 | 30 | 3 | 1 | 78,16 | 35,14 | 10,85 | 6,72 |
| 275 | 50 | 3 | 1 | 75,25 | 31,82 | 13,67 | 5,42 |
| 275 | 50 | 6 | 1 | 75,5 | 23,63 | 8,53 | 2,91 |
| 275 | 50 | 3 | 2 | 47,9 | 22,27 | 10,06 | 4,42 |
| 250 | 30 | 3 | 1 | 24,2 | 12,4 | 3,6 | 2,8 |
| ***300*** | ***30*** | ***3*** | ***1*** | ***100*** | ***5,80*** | ***33,3*** | ***12,8*** |

| | | | | | | | |
|---|---|---|---|---|---|---|---|
| (1): on entend par conversion le rapport de la différence entre le poids initial de la charge et le poids final de la charge au poids initial de la charge, exprimé en %. | | | | | | | |
| (2) : le rendement est le rapport du poids des produits obtenus au poids initial de la charge, exprimé en %. | | | | | | | |

Les deux lignes en caractères gras et italiques correspondent aux conditions opératoires les plus favorables, respectivement, pour l'isomérisation de la charge ou pour l'hydrogénolyse en produits contenant plus de 5 atomes de carbone.

Il a été constaté que le ratio i-paraffine/n-paraffine des différentes familles d'hydrocarbures augmente de manière significative lorsque la conversion, et donc le pouvoir hydrogénolysant du catalyseur, augmentent. Ce résultat signifie qu'avant de se craquer, un hydrocarbure adsorbé subit au moins une étape d'isomérisation.

Ce résultat est intéressant pour les produits entrant dans la composition des essences (C₅-C₁₁), puisque l'indice d'octane augmente avec le degré de ramification des paraffines. Par ailleurs, si les produits formés ne sont pas à priori intéressants pour constituer un gazole, ils le sont pour constituer la base d'huile de haute qualité à VI (indice de viscosité) élevé nécessitant des paraffines à longues chaînes ramifiées.

### 2- Transformation du n-dodécane

Il est bien connu que l'interaction des paraffines avec ce genre de catalyseur augmente avec la longueur de la chaîne carbonée. On peut donc s'attendre à une modification des propriétés du catalyseur de l'exemple 9 (Pt/Ferrierite) en présence de dodécane.

Les résultats obtenus indiquent, un profil de produits formés tout à fait comparable à celui obtenu avec l'utilisation de l'hexadécane comme réactif. Ici encore, l'isomérisation du réactif est favorisée avec l'utilisation de conditions douces de réaction (faible température, temps de séjour élevé, H₂/HC petit) tandis que l'hydrogénolyse de ce réactif dans les conditions plus sévères abouti plus favorablement à la formation de composés légers (C₁-C₄). L'obtention de composés intermédiaires de craquage (C₅-C₁₁) n'est jamais majoritaire dans nos conditions opératoires, mais représente une quantité non négligeable des produits formés.

A titre d'illustration, quelques résultats issus de la transformation du n-dodécane sur Pt/Ferrierite sont présentés dans le tableau IV.

**Tableau IV**

| **Hydrocraquage du n-C**_{**12**} **sur 0,8 % en poids Pt/FERRIERITE** | | | | | | |
|---|---|---|---|---|---|---|
| **T (°C)** | **P (bar)** | **H**_{**2**}**/HC** | **pph h**^{**-1**} | **Conversion (%)** | **Rendement iC12 (%)** | **Rendement essence (%)** |
| 300 | 30 | 3 | 1 | 79,04 | 15,14 | 35,96 |
| 300 | 50 | 3 | 1 | 85,21 | 14,98 | 38,01 |
| 300 | 50 | 6 | 1 | 79,95 | 11,01 | 31,40 |
| 280 | 50 | 6 | 0,75 | 77,48 | 20,01 | 39,49 |
| 280 | 20 | 6 | 1 | 70,26 | 24,94 | 42,92 |
| ***280*** | ***30*** | ***6*** | ***1*** | ***69,55*** | ***25, 64*** | ***41,20*** |
| 280 | 50 | 6 | 1 | 68,11 | 19,78 | 36,39 |

Ainsi, les résultats obtenus pour les réaction d'hydrocraquage et d'hydroisomérisation du dodécane et de l'hexadécane sur le catalyseur Pt/Ferrierite montrent que l'utilisation de conditions « douces » de réaction (température <280°C, H₂/HC ≤ 3, pph ≥ 1) favorise l'isomérisation des réactifs étudiés par rapport à leur hydrogénolyse. Dans ces conditions, la formation de produits intervenant dans la base des huiles de haute qualité (paraffines branchées) est favorisée (45% de rendement).

Par contre, lorsque la température de réaction augmente et/ou la valeur du rapport H₂/HC ou encore celle du pph diminue, l'obtention de produits plus légers que les réactifs devient prépondérante. Si la quantité de C₁-C₄ formée est importante autant lors de la transformation du dodécane que de l'hexadécane, les rendements en fractions essence (C₅-C₁₁) et gazole (C₁₂-C₁₅) sont intéressants (environ 45 % dans les meilleures conditions étudiées).

## Revendications

1. Procédé de préparation d'une zéolithe du type ferrierite, à taux de cristallinité élevé, et ayant une composition au stade anhydre exprimée par la formule Mₓ (AlO₂)ₓ (SiO₂)_{y}, M étant un cation d'un métal alcalin tel que Na ou K, ou un mélange des deux, **caractérisé en ce qu'** il comprend une étape d'obtention d'un gel, par addition d'acide borique à une solution aqueuse de sulfate d'aluminium et mélange avec une solution aqueuse contenant des oxydes de Na et/ou K et de Si, sans emploi d'agent structurant.

2. Procédé selon la revendication 1, **caractérisé en ce que** le mélange des solutions se fait sous agitation, avec acidification jusqu'à pH 8.

3. Procédé selon la revendication 2, **caractérisé en ce que** le gel obtenu est lavé à l'eau distillée .

4. Procédé selon la revendication 3, **caractérisé en ce qu'**une solution contenant des hydroxydes de métaux alcalins est incorporée sous agitation dans le mélange.

5. Procédé selon la revendication 4, **caractérisé en ce que** le gel obtenu est soumis à une étape de vieillissement, suivie d'une étape de cristallisation en autoclave, sous agitation et à chaud, pendant plusieurs heures.

6. Procédé selon la revendication 5, **caractérisé en ce que** le produit cristallisé obtenu est lavé à l'eau et séché à chaud.

7. Procédé selon l'une des revendications 1 à 4, **caractérisé en ce que** les étapes d'obtention du gel et d'adjonction des hydroxydes de métaux alcalins se déroulent à une température comprise entre 40 et 90°C.

8. Procédé selon l'une des revendications 5 à 7, **caractérisé en ce que** l'étape de vieillissement du gel est effectuée à une température comprise entre 25 et 80°C et pendant 6 à 72 heures.

9. Procédé selon l'une des revendications 5 à 8, **caractérisé en ce que** l'étape de cristallisation s'effectue à pression autogène, à une température comprise entre 150 et 200°C et pendant 2 à 72 heures.

10. Procédé selon l'une quelconque des revendications précédentes, **caractérisé en ce que** l'étape de cristallisation est suivie d'une calcination, d'une imprégnation avec une solution d'un sel de platine, puis d'une évaporation, d'un séchage et d'une autre calcination.

## Claims

1. A method of preparing a zeolite of the ferrierite type, having a high degree of crystallinity and having a composition at the anhydrous stage expressed by the formula Mₓ(AlO₂)ₓ(SiO₂)_{y}, M being a cation of an alkali metal such as Na or K, or a mixture of the two, **characterised in that** it comprises a step of obtaining a gel by addition of boric acid to an aqueous solution of aluminium sulphate and mixing with an aqueous solution containing oxides of Na and/or K and Si, without using a structuring agent.

2. A method according to claim 1, **characterised in that** the mixing of the solutions is carried out while stirring, with acidification up to pH 8.

3. A method according to claim 2, **characterised in that** the gel obtained is washed with distilled water.

4. A method according to claim 3, **characterised in that** a solution containing alkali metal hydroxides is incorporated into the mixture while stirring.

5. A method according to claim 4, **characterised in that** the gel obtained is subjected to an ageing step, followed by a crystallisation step in an autoclave, while stirring and in a heated state, for several hours.

6. A method according to claim 5, **characterised in that** the crystallised product obtained is washed with water and dried at a high temperature.

7. A method according to any one of claims 1 to 4, **characterised in that** the steps of obtaining the gel and adding alkali metal hydroxides take place at a temperature of between 40 and 90°C.

8. A method according to any one of claims 5 to 7, **characterised in that** the step of ageing the gel takes place at a temperature of between 25 and 80°C and for 6 to 72 hours.

9. A method according to any one of claims 5 to 8, **characterised in that** the crystallisation step takes place at autogenous pressure, at a temperature of between 150 and 200°C and for 2 to 72 hours.

10. A method according to any one of the preceding claims, **characterised in that** the crystallisation step is followed by calcination, impregnation with a solution of a platinum salt, then evaporation, drying and further calcination.

## Patentansprüche

1. Verfahren zur Herstellung eines Zeoliths des Ferrierit-Typs mit erhöhter Kristallinität, welches im anhydriden Stadium eine Zusammensetzung aufweist, das durch die allgemeine Formel Mₓ(AlO₂)ₓ(SiO₂)_{y} ausgedrückt wird, wobei M ein Kation eines Alkalimetalls wie Na oder K oder ein Gemisch aus diesen ist, **dadurch gekennzeichnet, dass** es einen Verfahrensschritt zum Erhalten eines Gels durch Zusatz von Borsäure zu einer wässrigen Lösung von Aluminiumsulfat und durch Vermischung mit einer wässrigen Lösung umfasst, welche Na- und/oder K-Oxide und Si enthält, ohne Verwendung eines Strukturierungsmittels.

2. Verfahren nach Anspruch 1, **dadurch gekennzeichnet, dass** die Vermischung der Lösungen unter Rühren mit Ansäuerung bis zu einem pH-Wert von 8 erfolgt.

3. Verfahren nach.Anspruch 2, **dadurch gekennzeichnet, dass** das erhaltene Gel mit destilliertem Wasser gewaschen wird.

4. Verfahren nach Anspruch 3, **dadurch gekennzeichnet, dass** eine Lösung, die Alkalimetall-Hydroxide enthält, unter Rühren in das Gemisch eingearbeitet wird.

5. Verfahren nach Anspruch 4, **dadurch gekennzeichnet, dass** das erhaltene Gel einem Verfahrensschritt zur Alterung mit anschließendem Verfahrensschritt zur Kristallisierung im Autoklaven unter Rühren und im heißen Zustand über mehrere Stunden unterzogen wird.

6. Verfahren nach Anspruch 5, **dadurch gekennzeichnet, dass** das erhaltene kristallisierte Produkt mit Wasser gewaschen und in der Wärme getrocknet wird.

7. Verfahren nach einem der Ansprüche 1 bis 4, **dadurch gekennzeichnet, dass** die Verfahrensschritte zum Erhalten des Gels und der Zugabe von Alkalimetall-Hydroxiden bei einer Temperatur zwischen 40 und 90 °C ablaufen.

8. Verfahren nach einem der Ansprüche 5 bis 7, **dadurch gekennzeichnet, dass** der Verfahrensschritt zur Alterung des Gels bei einer Temperatur zwischen 25 und 80 °C über 6 bis 72 Stunden abläuft.

9. Verfahren nach einem der Ansprüche 5 bis 8, **dadurch gekennzeichnet, dass** der Verfahrensschritt zur Kristallisierung unter autogenem Druck bei einer Temperatur zwischen 150 und 200 °C über 2 bis 72 Stunden abläuft.

10. Verfahren nach einem der vorhergehenden Ansprüche, **dadurch gekennzeichnet, dass** sich an den Verfahrensschritt zur Kristallisierung eine Kalzinierung, eine Imprägnierung mit einer Lösung eines Platinsalzes, dann eine Verdampfung, eine Trocknung und eine weitere Kalzinierung anschließen.
